# EUROPEAN PATENT APPLICATION

(11) **EP 3 217 164 A1**
(43) Date of publication of application: **13.09.2017**
(21) Application number: 15857473.1
(22) Date of filing: 26.10.2015
(51) Int. Cl.: G01N 21/64

(54) **DETECTION DEVICE AND DETECTION METHOD**

(30) Priority: 07.11.2014 JP 2014227031
(71) Applicant: Konica Minolta, Inc., Tokyo 100-7015 (JP)
(72) Inventor: OOTANI, Makiko, Tokyo 100-7015 (JP); TAMURA, Tsuruki, Tokyo 100-7015 (JP)
(74) Representative: Gille Hrabal
(86) International application number: PCT/JP2015/080144
(87) International publication number: WO 2016/072307

(57) **Abstract**

A detection device is provided with a holder, light irradiation unit, signal detection unit, and processing unit. The holder holds a detection chip. The light irradiation unit irradiates light onto the detection chip held by the holder. When the light irradiation unit irradiates the detection chip with light, the signal detection unit detects, at least twice, a signal generated at a reaction site. The processing unit calculates a signal value variation rate on the basis of a first signal value detected by the signal detection unit and a second signal value detected after the first signal value.

## Description

### Technical Field

The present invention relates to a detection apparatus and a detection method for detecting an analyte in a sample.

### Background Art

In a clinical test or the like, highly sensitive and quantitative detection of a trace amount of analyte, such as a protein or DNA, in a sample, would allow a quick understanding of patient's condition and the subsequent his/her treatment. For this reason, there is a need for a method of detecting an analyte in a sample highly sensitively and quantitatively.

In order to analyze a trace amount of analyte in a sample, an immunoassay is performed. In an immunoassay, a sample is generally provided to a reaction site, such as a magnetic particle or a planar substrate, in which a capturing body (antibody) that specifically captures an analyte is immobilized to allow the analyte to bind to the capturing body specifically (also referred to as "primary reaction" hereinafter). Then, the analyte captured by the capturing body is labeled with a labeling substance (e.g., a fluorophore, a radioisotope), a signal from the labeling substance is detected, and thus the analyte can be detected.

Despite the absence of an analyte in a reaction site, however, the detected value sometimes indicates positive (referred to as "false positive"). Thus, in order to conduct a detailed analysis of such specific binding of a biomolecule or the like, various types of analyzing systems have been developed in recent years (see, Patent Literature (hereinafter, abbreviated as PTL) 1, for example).

The analysis sensor system described in PTL 1 can determine the concentration of a binding partner (analyte) in a solution, which can reversibly interact with a ligand (capturing body) immobilized on a support surface (sensor surface). In the analysis sensor system, solutions containing a binding partner in various known concentrations (samples) are first allowed to flow on the support surface so that the binding partner and the ligand bind to each other (step a). Then, a binding partner-free solution is allowed to flow on the support surface so that the binding partner dissociates from the ligand (step b). The analysis sensor system collects data, between step a and step b, by monitoring an instantaneous amount of the binding partner bound to the support surface (step c). Then, a binding rate constant, a dissociation rate constant, and the like can be determined by fitting the collected date to a predetermined kinetic model (step d).

### Citation List

### Patent Literature

PTL 1
Japanese Unexamined Patent Application Publication (Translation of PCT Application) No. 2006-527365

### Summary of Invention

### Technical Problem

As described above, in the detection of analytes, the detected values sometimes indicate false positive. This is caused by a phenomenon in which substances other than analytes in samples are adsorbed nonspecifically to reaction sites and detected as signals. In order to judge whether detected signals are derived from analytes or from substances other than analytes, a new additional apparatus or a separate test (measurement) is needed. This leads to delay in detection time as well as increase in detection costs and manufacturing costs of the detection apparatus.

Meanwhile, although the analysis sensor system described in PTL 1 can perform a detailed analysis of, for example, binding rates and dissociation rates of biomolecules, it cannot be used for the detection of analytes since the system needs to know the concentrations of analytes in solutions in advance. Further, there has been no disclosure about the detection of analytes using a binding rate, a dissociation rate, and the like.

An object of the present invention is to provide a detection apparatus and a detection method that can obtain information for judging whether detected signals are derived from analytes or not without adding a new apparatus or a measurement step.

### Solution to Problem

To achieve at least one of the above-mentioned objects, a detection apparatus according to an embodiment of the present invention configured to detect an analyte in a sample using a detection chip having a reaction site where a capturing body is immobilized, including: a holder for holding the detection chip; a light irradiation section configured to irradiate the detection chip held by the holder with light; a signal detection section configured to detect at least twice a signal generated in the reaction site when the light irradiation section irradiates the detection chip with light; and a processing section configured to calculate a change rate of signal values based on a first signal value detected by the signal detection section and a second signal value detected after the first signal value.

To achieve one of the above-mentioned objects, a detection method according to an embodiment of the present invention for detecting an analyte in a sample using a detection chip having a reaction site where a capturing body is immobilized, including: providing the sample to the reaction site of the detection chip; obtaining a first signal value by irradiating the detection chip with light in a state in which the sample provided to the reaction site is removed, and detecting a signal generated in the reaction site; obtaining a second signal value after obtaining the first signal value by irradiating the detection chip with light and detecting a signal generated in the reaction site; and calculating a change rate of signal values based on the first signal value and the second signal value.

### Advantageous Effects of Invention

According to the present invention, detected results about the presence or the amount of analytes, as well as information for judging the reliability of the detected results can be obtained without adding a new apparatus. Thus, according to the present invention, analytes can be detected highly reliably in a short time at a low cost.

### Brief Description of Drawings

FIG. 1 is a schematic view illustrating a configuration of surface plasmon-field enhanced fluorescence spectroscopy apparatus (SPFS apparatus) according to an embodiment;
FIG. 2 is a flow chart illustrating an operational procedure at SPFS apparatus according to an embodiment;
FIG. 3 is a graph for explaining an operational mechanism at SPFS apparatus according to an embodiment;
FIG. 4 is a schematic view illustrating an example of the content to be output in an output section;
FIG. 5 is a histogram illustrating the distribution of reduction rates in quantity of light for fluorescence when signal values were detected using cTnI samples and HAMA samples; and
FIG. 6 is a graph illustrating reduction rates in quantity of light for fluorescence when signal values were detected using cTnI samples and HAMA samples.

### Description of Embodiments

Embodiments of the present invention will be described in detail hereinafter with reference to the accompanying drawings. As a representative example of a detection apparatus according to the present invention, a detection apparatus (SPFS apparatus) based on surface plasmon-field enhanced fluorescence spectroscopy (abbreviated as "SPFS" hereinafter) will be described. The SPFS apparatus according to the embodiment detects signal values that indicate the presence or the amount of an analyte, performs such signal detection at least twice, and thus can obtain information about the reliability of the signal values that indicate the presence or the amount of an analyte, based on a change rate of two or more signal values obtained.

### [Configurations of SPFS apparatus and Detection Chip]

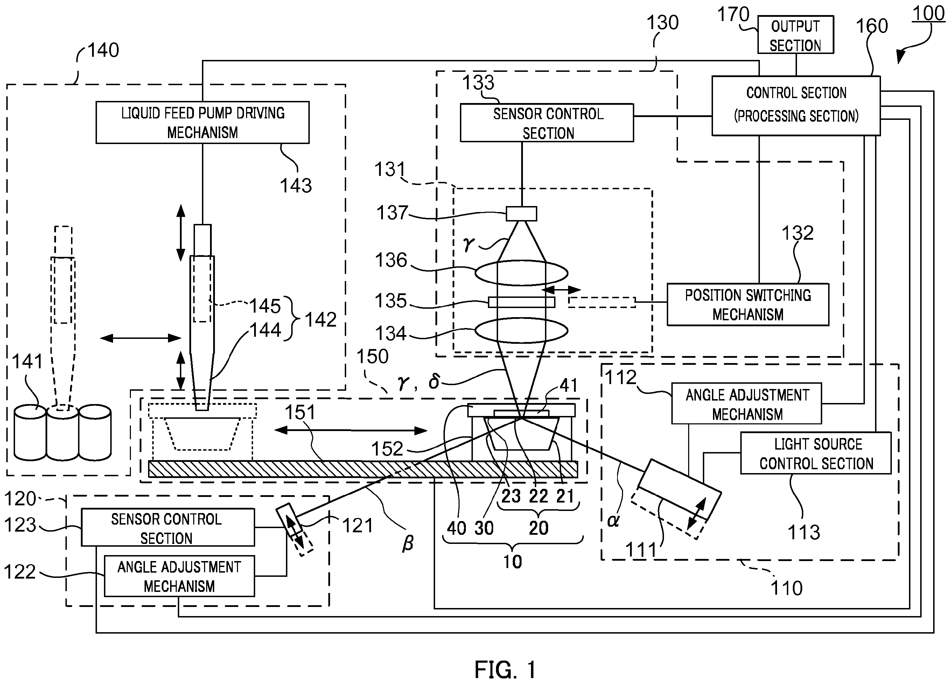

FIG. 1 is a schematic view illustrating a configuration of SPFS apparatus 100. SPFS apparatus 100 includes light irradiation section 110, reflected light detection section 120, signal detection section 130, liquid feed section 140, conveyance section 150, control section 160, and output section 170. SPFS apparatus 100 is used in a state in which detection chip 10 is mounted on chip holder 152 in conveyance section 150. Detection chip 10 will be described first, and then each component of SPFS apparatus will be described later.

Detection chip 10 includes: prism 20 having incident surface 21, film forming surface 22, and emission surface 23; metal film 30 formed on film forming surface 22; capturing bodies immobilized in a region of metal film 30 that becomes a reaction site; and channel lid 40 disposed on metal film 30. In the reaction site, binding of capturing bodies and analytes (primary reaction), labeling of analytes with fluorescent substances (secondary reaction), or the like occurs. Further, detection chip 10 is generally replaced for each detection. Detection chip 10 is preferably a structure having each side length of a few millimeters to a few centimeters, but may be a smaller structure or a larger structure excluded from a category of "chip."

Prism 20 is made of a dielectric transparent to excitation light α. Prism 20 includes incident surface 21, film forming surface 22, and emission surface 23. Incident surface 21 is a surface through which excitation light α from light irradiation section 110 enter prism 20. On film forming surface 22, metal film 30 is disposed. Excitation light α entering inside prism 20 is reflected on a rear surface of metal film 30 to be reflected light β. More specifically, excitation light α is reflected at the interface (film forming surface 22) between prism 20 and metal film 30 and forms reflected light β. Emission surface 23 emits reflected light β outside prism 20.

The shape of prism 20 is not limited. In the embodiment, the shape of prism 20 is a prism having trapezoidal bases. The surface corresponding to either the base of a trapezoid is film forming surface 22, the surface corresponding to either the legs is incident surface 21, and the surface corresponding to the other leg is emission surface 23. A trapezoid as a base is preferably an isosceles trapezoid. This makes incident surface 21 and emission surface 23 symmetrical, and hinders confinement of s-wave component of excitation light α inside prism 20.

Incident surface 21 is formed so that excitation light α does not return to light irradiation section 110. When a light source of excitation light α is a laser diode (also referred to as "LD" hereinafter), returned excitation light α to LD would disturb the excitation state of LD and alter the wavelength and/or the output of excitation light α. Thus, the angle of incident surface 21 is set within an ideal scanning range centered on a resonance angle or an enhanced angle so that excitation light α does not enter incident surface 21 perpendicularly. The term "resonance angle" herein refers to an incident angle in which quantity of light for reflected light β emitted from emission surface 23 becomes minimum during scanning over incident angles of excitation light α on metal film 30. In contrast, the term "enhanced angle" herein refers to an incident angle in which quantity of light for scattered light δ with the same wavelength as excitation light α emitted above detection chip 10 (referred to as "plasmon scattered light" hereinafter) becomes maximun during scanning over incident angles of excitation light α on metal film 30. In the embodiment, both the angle between incident surface 21 and film forming surface 22, and the angle between film forming surface 22 and emission surface 23 are about 80°.

A resonance angle (and an enhanced angle in the immediate vicinity thereof) is largely determined by the design of detection chip 10. Such design factors include a refractive index of prism 20, a refractive index of metal film 30, a thickness of metal film 30, an extinction coefficient of metal film 30, a wavelength of excitation light α, and the like. Although a resonance angle and an enhanced angle are shifted by analytes captured on metal film 30, the shift is less than a few degrees.

Prism 20 exhibits at least birefringence characteristics. Examples of materials for prism 20 include resins and glass. The materials for prism 20 are preferably resins having a refractive index of 1.4 to 1.6 and a small birefringence.

Metal film 30 is disposed on film forming surface 22 of prism 20. This can generate surface plasmon resonance (abbreviated as "SPR" hereinafter) between photons of excitation light α incident on film forming surface 22 under total reflection conditions and free electrons of metal film 30, and thus generate localized-field light (also generally called as "evanescent light" or "near-field light"). In the embodiment, metal film 30 is formed on the whole film forming surface 22.

Materials for metal film 30 are not limited as long as metals can generate surface plasmon resonance. Examples of the materials for metal film 30 include gold, silver, copper, aluminum, and an alloy thereof. In the embodiment, metal film 30 is a metal film. The method for forming metal film 30 is not limited. Examples of the method for forming metal film 30 include sputtering, vapor deposition, and plating. The thickness of metal film 30 is preferably, but not limited to, 30 to 70 nm.

Further, capturing bodies (not shown) for capturing analytes are immobilized on a surface of metal film 30 facing away from prism 20 (surface of metal film 30). In the embodiment, the capturing bodies are evenly immobilized in a predetermined region (reaction site) above metal film 30. The types of the capturing bodies are not limited as long as they capture analytes. In the embodiment, the capturing bodies are antibodies or fragments thereof that specifically bind to analytes. From the viewpoint of preventing denaturation due to drying, capturing bodies are generally stored using a protective layer before detection chip 10 is used.

Channel lid 40 is disposed on metal film 30. When metal film 30 is formed in only part of film forming surface 22 of prism 20, channel lid 40 may be disposed on film forming surface 22. In the embodiment, channel lid 40 is disposed on metal film 30. Channel grooves are formed on the rear surface of channel lid 40, and channel lid 40, together with metal film 30 (and prism 20), forms channel 41 that retains a liquid and allows the liquid to flow. Further, a reaction site is disposed to be exposed to channel 41. This means that capturing bodies immobilized above metal film 30 are also exposed to inside channel 41. Examples of the liquid include a sample containing an analyte (e.g., blood, serum, plasma, urine, nostril mucus, saliva, semen), a labeling solution containing capturing bodies labeled with a fluorescent substance, and a rinse solution. Both ends of channel 41 are respectively connected to an inlet and an outlet (not shown) formed on the top surface of channel lid 40. When a liquid is provided to inside channel 41, the liquid comes into contact with capturing bodies.

Channel lid 40 is preferably formed from a material transparent to fluorescence γ emitted from above metal film 30 and plasmon scattered light δ. Examples of materials for channel lid 40 include resins. As long as a portion, through which fluorescence γ and plasmon scattered light δ is extracted outside, is transparent to fluorescence γ and plasmon scattered light δ, other portions of channel lid 40 may be formed from opaque materials.Channel lid 40 is joined with metal film 30 or prism 20, for example, through bonding using a double-stick tape, an adhesive, or the like, laser welding, ultrasonic welding, or pressure bonding using a clamping member.

Instead of channel lid 40, detection chip 10 may include a frame having a through hole and a top plate to be stacked on the frame. In this case, channel 41 is formed by stacking the frame and the top plate in this order on prism 20, on which metal film 30 has been formed. The surface of metal film 30 constitutes a bottom surface of channel 41. The inner surface of the through hole of the frame constitutes a side surface of channel 41. One surface of the top plate (inner surface) constitutes a top surface of channel 41.

As illustrated in FIG. 1, excitation light α enters prism 20 at incident surface 21. Excitation light α that has entered prism 20 is incident on metal film 30 at a total reflection angle (SPR-generating angle). Irradiation of metal film 30 with excitation light α at such a SPR-generating angle can generate localized-field light above metal film 30. The localized-field light excites a fluorescent substance that labels an analyte present above metal film 30, and fluorescence γ is emitted from the vicinity of a surface of metal film 30 on the side of channel 41. SPFS apparatus 100 detects the presence or the amount of an analyte by measuring quantity of light for fluorescence γ emitted from the fluorescent substance.

Each component of SPFS apparatus will now be described. As described above, SPFS apparatus 100 includes light irradiation section 110, reflected light detection section 120, signal detection section 130, liquid feed section 140, conveyance section 150, control section 160, and output section 170.

Light irradiation section 110 irradiates detection chip 10 held on chip holder 152 with excitation light α. When fluorescence γ or plasmon scattered light δ is detected, light irradiation section 110 emits only p-wave (relative to metal film 30) on incident surface 21 so that an incident angle on metal film 30 becomes a SPR-generating angle. The term "excitation light" herein refers to light that excites a fluorescent substance directly or indirectly. For example, excitation light α is light that generates, above the surface of metal film 30, localized-field light that excites a fluorescent substance when irradiated on metal film 30 through prism 20 at a SPR-generating angle. Light irradiation section 110 includes light source unit 111, angle adjustment mechanism 112, and light source control section 113.

Light source unit 111 emits excitation light α, which is collimated and has a certain wavelength and quantity of light, so that the shape of an irradiation spot on a rear surface of metal film 30 becomes almost circular. Light source unit 111 includes, for example, a light source of excitation light α, a beam shaping optical system, an APC mechanism, and a temperature adjustment mechanism (neither shown).

The light source, whose type is not limited, is a laser diode (LD), for example. Other examples of the light source include a light-emitting diode, a mercury lamp, and other laser light sources. When light emitted from the light source is not a beam, it is converted to a beam by a lens, a mirror, a slit, or the like. Also, when light emitted from the light source is not monochromatic light, it is converted to monochromatic light by a diffraction grating or the like. Further, when light emitted from the light source is not linearly polarized light, it is converted to linearly polarized light by a polarizer or the like.

The beam shaping optical system includes, for example, a collimator, a bandpass filter, a linear polarizing filter, a half-wave plate, a slit, and/or a zoom unit. The beam shaping optical system may include all of them or some of them. The collimator collimates excitation light α emitted from the light source. The bandpass filter converts excitation light α emitted from the light source to narrow-band light only composed of the central wavelength of excitation light α. This is done because excitation light α from the light source has some wavelength distribution widths. The linear polarizing filter converts excitation light α emitted from the light source to completely linearly polarized light. The half-wave plate adjusts the polarization direction of excitation light α so that p-wave component is incident on metal film 30. The slit and zoom unit adjust the beam diameter or the contour shape of excitation light α so that the shape of an irradiation spot on the rear surface of metal film 30 becomes circular of predetermined size.

The APC mechanism controls the light source so that the output of the light source becomes constant. More specifically, the APC mechanism detects quantity of light for light split from excitation light α with a photodiode or the like (not shown). Then, the APC mechanism controls the output of the light source to become constant by controlling an input energy with a feedback circuit.

The temperature adjustment mechanism is a heater or a Peltier device, for example. The wavelength and the energy of emission light from the light source may fluctuate depending on the temperature. For this reason, the wavelength and the energy of emission light from the light source are controlled to become constant by maintaining a constant temperature of the light source with the temperature adjustment mechanism.

Angle adjustment mechanism 112 adjusts the incident angle of excitation light α on metal film 30 (interface (film forming surface 22) between prism 20 and metal film 30). Angle adjustment mechanism 112 relatively rotates the optical axis of excitation light α and chip holder 152 in order that excitation light α is irradiated in a predetermined position of metal film 30 through prism 20 at a predetermined incident angle.

For example, angle adjustment mechanism 112 turns light source unit 111 around an axis orthogonal to the optical axis of excitation light α (axis perpendicular to the plane of FIG. 1). In this operation, the position of the rotational axis is set so that the position of an irradiation spot on metal film 30 scarcely changes even during scanning over incident angles. In particular, the displacement of the irradiation position can be minimized by setting the position of the rotation center to the vicinity of an intersection of optical axes of excitation light α at both ends of the scanning range over incident angles (between the irradiation position on film forming surface 22 and incident surface 21).

As described above, an angle in which quantity of light for plasmon scattered light δ becomes maximum is an enhanced angle, among incident angles of excitation light α on metal film 30. By setting the incident angle of excitation light α to an enhanced angle or an angle in the vicinity thereof, it is possible to detect high-intensity fluorescence γ. Although basic incidence conditions of excitation light α are determined by a material and the shape of prism 20 of detection chip 10, a thickness of metal film 30, a refractive index of a liquid inside channel 41, and the like, optimal incidence conditions slightly fluctuate depending on the type and the amount of a fluorescent substance inside channel 41, an error in the shape of prism 20, or the like. Thus, it is preferable to obtain an optimal enhanced angle for each detection.

Light source control section 113 controls emission of excitation light α from light source unit 111 by controlling various types of equipment included in light source unit 111. Light source control section 113 is configured, for example, as a commonly known computer or a microcomputer including an arithmetic apparatus, a control apparatus, a storage apparatus, an input apparatus, and an output apparatus.

In order to measure a resonance angle or the like, reflected light detection section 120 measures quantity of light for reflected light β generated by irradiation of detection chip 10 with excitation light α. Reflected light detection section 120 includes light receiving sensor 121, angle adjustment mechanism 122, and sensor control section 123.

Light receiving sensor 121 is disposed in an incident position of reflected light β and measures quantity of light for reflected light β. The type of light receiving sensor 121 is not limited. Light receiving sensor 121 is a photodiode (PD), for example.

Angle adjustment mechanism 122 adjusts the position (angle) of light receiving sensor 121 in accordance with an incident angle of excitation light α on metal film 30. Angle adjustment mechanism 122 relatively rotates light receiving sensor 121 and chip holder 152 so that reflected light β is incident on light receiving sensor 121.

Sensor control section 123 controls, for example, detection of output values at light receiving sensor 121, sensitivity regulation at light receiving sensor 121 using detected output values, and changing in sensitivity at light receiving sensor 121 to obtain proper output values. Sensor control section 123 is configured, for example, as a commonly known computer or a microcomputer including an arithmetic apparatus, a control apparatus, a storage apparatus, an input apparatus, and an output apparatus.

Signal detection section 130 detects at least twice fluorescence γ (signal) generated in a reaction site upon irradiation of excitation light α on metal film 30 by light irradiation section 110. In the embodiment, the number of the detection of fluorescence γ is twice. As needed, signal detection section 130 also detects plasmon scattered light δ generated in a reaction site upon irradiation of excitation light α on metal film 30 by light irradiation section 110. Signal detection section 130 includes light receiving unit 131, position switching mechanism 132, and sensor control section 133.

Light receiving unit 131 is disposed in the normal direction to metal film 30 of detection chip 10. Light receiving unit 131 includes first lens 134, optical filter 135, second lens 136, and light receiving sensor 137.

First lens 134 is, for example, a condensing lens, and focuses light emitted from above metal film 30. Second lens 136 is, for example, an imaging lens, and forms an image of light focused by first lens 134 on a light receiving surface of light receiving sensor 137. The optical paths between the lenses are almost parallel. Optical filter 135 is disposed between the lenses.

Optical filter 135 only guides a fluorescent component to light receiving sensor 137 while removing an excitation light component (plasmon scattered light δ) to detect fluorescence γ at a high S/N ratio. Examples of optical filter 135 include an excitation light-reflective filter, a short wavelength-blocking filter, and a bandpass filter. Optical filter 135 is, for example, a filter containing a multilayer film that reflects a predetermined light component (light with a predetermined wavelength component) or a color glass filter that absorbs a specific light component.

Light receiving sensor 137 detects fluorescence γ and plasmon scattered light δ. Light receiving sensor 137 has a high sensitivity so that faint fluorescence γ from a trace amount of analyte is detected. Light receiving sensor 137 is a photomultiplier tube (PMT) or an avalanche photodiode (APD), for example.

Position switching mechanism 132 switches the position of optical filter 135 between on the optical path and off the optical path of light receiving unit 131. Specifically, when light receiving sensor 137 detects fluorescence γ, optical filter 135 is disposed on the optical path of light receiving unit 131, whereas when light receiving sensor 137 detects plasmon scattered light δ, optical filter 135 is disposed off the optical path of light receiving unit 131.

Sensor control section 133 controls, for example, detection of output values at light receiving sensor 137, sensitivity regulation at light receiving sensor 137 using detected output values, and changing in sensitivity at light receiving sensor 137 to obtain proper output values. Sensor control section 133 is configured, for example, as a commonly known computer or a microcomputer including an arithmetic apparatus, a control apparatus, a storage apparatus, an input apparatus, and an output apparatus.

Liquid feed section 140 supplies a sample, a labeling solution, a rinse solution, or the like to inside channel 41 of detection chip 10 held on chip holder 152. Liquid feed section 140 includes liquid chips 141, syringe pump 142, and liquid feed pump driving mechanism 143.

Liquid chips 141 are containers that retain a liquid, such as a sample, a labeling solution, or a rinse solution. As liquid chips 141, in general, a plurality of containers are disposed in accordance with the types of liquids, or a plurality of containers are disposed as an integrated chip.

Syringe pump 142 is composed of syringe 144 and plunger 145 which can make reciprocating motion inside syringe 144. By reciprocating movements of plunger 145, liquids are quantitatively sucked and discharged. When syringe 144 is replaceable, cleaning of syringe 144 is not required. This is preferable from the viewpoint of preventing contamination by impurities and the like. When syringe 144 is not configured to be replaceable, further addition of a component for cleaning inside syringe 144 would allow the use of syringe 144 without replacing.

Liquid feed pump driving mechanism 143 includes a driving apparatus for plunger 145 and a moving apparatus for syringe pump 142. The driving apparatus for plunger 145 is an apparatus for moving plunger 145 reciprocally, and includes a stepping motor, for example. Since a driving apparatus including a stepping motor can regulate a liquid feed volume or a liquid feed rate of syringe pump 142, such a driving apparatus is preferable from the viewpoint of regulating a residual liquid volume of detection chip 10. The moving apparatus for syringe pump 142, for example, freely moves syringe pump 142 both in the axial direction of syringe 144 (e.g., vertical direction) and in the direction crossing the axial direction (e.g., horizontal direction). The moving apparatus for syringe pump 142 is configured, for example, as a robot arm, a two-axis stage, or vertically movable turntable.

Liquid feed section 140 sucks various liquids from liquid chips 141 and supplies them to inside channel 41 of detection chip 10. During this operation, liquids are moved reciprocally inside channel 41 of detection chip 10 by moving plunger 145, and thus the liquids inside channel 41 are stirred. This can achieve uniform concentration distribution of liquids, promoted reactions inside channel 41 (e.g., primary reaction and secondary reaction described hereinafter), and the like. From the viewpoint of performing such operation, it is preferable to configure detection chip 10 and syringe 144 so that an inlet of detection chip 10 is protected with a multilayer film, and the inlet is sealed when syringe 144 penetrates this multilayer film.

A liquid inside channel 41 is sucked again with syringe pump 144 and discharged into liquid chips 141 and the like. Through repeating this operation, reactions by various liquids, cleaning, and the like are carried out, thereby disposing an analyte labeled with a fluorescent substance in a reaction site inside channel 41.

Conveyance section 150 conveys detection chip 10 to an installation position, a detection position, or a liquid feed position, and fixes it. The term "installation position" herein refers to a position for installing detection chip 10 in SPFS apparatus 100. The term "detection position" herein refers to a position in which reflected light detection section 120 or signal detection section 130 detects reflected light β, fluorescence γ, or plasmon scattered light δ generated upon irradiation of excitation light α on detection chip 10 by light irradiation section 110. Further, the term "liquid feed position" herein refers to a position in which liquid feed section 140 supplies a liquid to inside channel 41 of detection chip 10 or removes a liquid inside channel 41 of detection chip 10. Conveyance section 150 includes conveyance stage 151 and chip holder 152. Chip holder 152 is fixed on conveyance stage 151 and holds detection chip 10 detachably. The shape of chip holder 152 is a shape that can hold detection chip 10 without obstructing the optical paths of excitation light α, reflected light β, fluorescence γ, and plasmon scattered light δ. For example, chip holder 152 has openings for passing excitation light α, reflected light β, fluorescence γ, and plasmon scattered light δ through. Conveyance stage 151 moves chip holder 152 in one direction and in the opposite direction. Conveyance stage 151 also has a shape that does not obstruct the optical paths of excitation light α, reflected light β, fluorescence γ, and plasmon scattered light δ. Conveyance stage 151 is driven by a stepping motor, for example.

Control section 160 controls angle adjustment mechanism 112, light source control section 113, angle adjustment mechanism 122, sensor control section 123, position switching mechanism 132, sensor control section 133, liquid feed pump driving mechanism 143, conveyance stage 151, and output section 170 (described hereinafter). Control section 160 also functions as a processing section that calculates a change rate of signal values based on detected results in signal detection section 130. Control section 160 is configured, for example, as a commonly known computer or a microcomputer including an arithmetic apparatus, a control apparatus, a storage apparatus, an input apparatus, and an output apparatus. Although the details will be described when the detection operation of SPFS apparatus 100 will be described, control section 160 may or may not store, in advance, a threshold value used for obtaining information about the reliability of signal values. In the embodiment, a storage apparatus of control section 160 stores a predetermined threshold value in advance.

Information obtained by the detection, information input by a user, and the like are output in output section 170. Output section 170 is, for example, a display, a printer, or the like. Examples of information obtained by the detection include output values at light receiving sensor 137 and calculation results in control section 160 (processing section). Further, examples of information input by a user include the type of an analyte, the amount of an analyte in a sample, a detection date and time, information about patients, and the like.

### [Detection Operation at SPFS Apparatus]

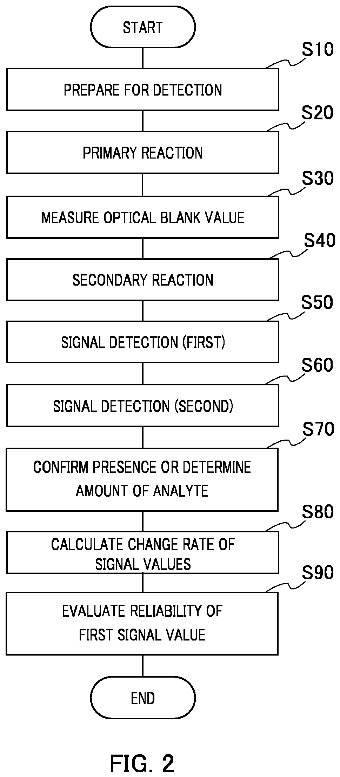

In the following, the detection operation at SPFS apparatus according to the embodiment (detection method according to the embodiment of the present invention) will be described. In the embodiment, SPFS apparatus 100 detects a signal twice and calculates a change rate of the obtained two signal values (first signal value and second signal value). Based on the calculation result, SPFS apparatus 100 detects a first signal value that indicates the presence or the amount of an analyte, and obtains information about the reliability of the first signal value. FIG. 2 is a flow chart illustrating an operational procedure at SPFS apparatus 100.

First, the detection is prepared. (step S 10). Specifically, detection chip 10 is installed in chip holder 152 disposed at an installation position of SPFS apparatus. Further, when a humectant is present inside channel 41 of detection chip 10, inside channel 41 is washed to remove the humectant so that a capturing body properly captures an analyte.

Then, the capturing body in a reaction site is allowed to capture the analyte (primary reaction; step S 20). Specifically, control section 160 moves detection chip 10 from the installation position to a liquid feed position by operating conveyance stage 151. Then, control section 160 provides a sample in liquid chips 141 to the reaction site inside channel 41 by operating liquid feed pump driving mechanism 143. In the primary reaction, the analyte and the capturing body bind to each other specifically. Meanwhile, a sample may contain not only the analyte, but also a substance other than the analyte. As a result, specific binding of the analyte and nonspecific adsorption of the substance other than the analyte to the capturing body, to metal film 30, or to an inner wall of channel 41 may occur simultaneously. Specific binding of the analyte and nonspecific adsorption of the substance other than the analyte are reversible reactions, and consequently dissociation as a reverse reaction also occurs. Over time, the specific binding, nonspecific adsorption, and dissociation reach equilibrium.

After that, control section 160 removes a sample provided to the reaction site and washes inside channel 41 at least once using a rinse solution (e.g., phosphoric acid buffer) by operating liquid feed pump driving mechanism 143.

Then, an optical blank value is measured (step S 30). The term "optical blank value" herein refers to quantity of light for background light emitted above detection chip 10 in the steps of detecting signal values described hereinafter (step S 50 and step S 60). Specifically, control section 160 moves detection chip 10 from the installation position to the liquid feed position by operating conveyance stage 151. After that, control section 160 irradiates a rear surface of metal film 30, corresponding to a region where the capturing body is immobilized, with excitation light α through incident surface 21 so as to generate SPR, and simultaneously records an output value at light receiving sensor 137 (optical blank value) by operating light irradiation section 110 and signal detection section 130. During this step, control section 160 sets an incident angle of excitation light α to an enhanced angle by operating angle adjustment mechanism 112. In addition, control section 160 disposes optical filter 135 on the optical path of light receiving unit 131 by controlling position switching mechanism 132. The detected optical blank value is recorded in control section 160.

Then, the analyte inside channel 41 is labeled with a fluorescent substance (secondary reaction; sept S 40). Specifically, control section 160 provides a liquid containing a capturing body labeled with the fluorescent substance (labeling solution) to inside channel 41 of detection chip 10 by operating liquid feed pump driving mechanism 143. Inside channel 41, the analyte is labeled with the fluorescent substance. During this step, the substance other than the analyte may also be labeled with the fluorescent substance by nonspecific adsorption.

After that, control section 160 removes the labeling solution inside channel 41 and washes inside channel 41 with a rinse solution by operating liquid feed pump driving mechanism 143.

Then, a signal generated in the reaction site above metal film 30 is detected and a first signal value that indicates the presence or the amount of the analyte is obtained (step S 50). Specifically, control section 160 moves detection chip 10 from the liquid feed position to the detection position by operating conveyance stage 151. During this step, the detection of the first signal value becomes possible when inside channel 41 is washed after the secondary reaction and detection chip 10 is moved to the detection position. Then, control section 160 irradiates a rear surface of metal film 30, corresponding to a region where the capturing body is immobilized, with excitation light α through incident surface 21 so as to generate SPR by operating light irradiation section 110, and simultaneously detects quantity of light for fluorescence γ by operating signal detection section 130. Control section 160 obtains the first signal value by subtracting the optical blank value from the detected quantity of light for fluorescence γ. In step S 50, control section 160 sets an incident angle of excitation light α to an enhanced angle by operating angle adjustment mechanism 112. In addition, control section 160 disposes optical filter 135 on the optical path of light receiving unit 131 by controlling position switching mechanism 132. Although the details will be described hereinafter, the first signal value is preferably detected within 3 minutes, more preferably within 30 seconds after a signal becomes detectable.

Then, a signal generated in the reaction site above metal film 30 is further detected to obtain a second signal value (step S 60). Specifically, control section 160 irradiates a rear surface of metal film 30, corresponding to a region where the capturing body is immobilized, with excitation light α through incident surface 21 so as to generate SPR by operating light irradiation section 110, and simultaneously detects quantity of light for fluorescence γ by operating signal detection section 130. Control section 160 obtains the second signal value by subtracting the optical blank value from the detected quantity of light for fluorescence γ. Although the details will be described hereinafter, the interval between obtaining the first signal value and obtaining the second signal value is preferably 20 to 60 seconds.

Then, based on the first signal value, the presence of the analyte is confirmed or the amount of the analyte is determined (step S 70). Specifically, control section 160 confirms the presence of the analyte when the first signal value is higher than or equal to a predetermined amount. In addition, control section 160 determines the amount of the analyte in a sample based on the first signal value and a prepared calibration curve representing a relationship between the quantity of light for fluorescence and the amount of the analyte.

Then, a change rate of signal values is calculated (step S 80). Specifically, control section 160 calculates a reduction rate in quantity of light for fluorescence γ based on the first signal value and the second signal value detected in step S 50 and step S 60, respectively.

Then, information about the reliability of the first signal value is obtained and whether the detected signals are derived from the analyte or not is judged (step S 90). Specifically, control section 160 obtains information about the reliability of the first signal value by comparing the change rate calculated in step S 80 (reduction rate in quantity of light for fluorescence) with a predetermined threshold value. After that, control section 160 judges whether the detected first signal value is derived from the analyte (whether the reliability is high) or is derived from the substance other than the analyte (whether the reliability is low) based on information about the reliability of the obtained first signal value. Information about the reliability of the first signal value is, for example, but not limited to, information about an effect of noise due to the substance other than the analyte adsorbed to the reaction site nonspecifically.

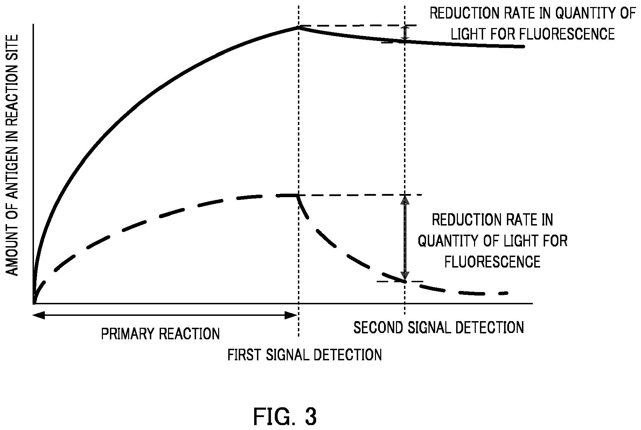

The method for obtaining information about the reliability of the first signal value will now be described in detail with reference to a graph. FIG. 3 is a graph for explaining an operational mechanism at SPFS apparatus 100. In FIG. 3, the horizontal axis represents time and the vertical axis represents the amounts of a specifically binding analyte and a nonspecifically adsorbing substance other than the analyte. In FIG. 3, the solid line represents the analyte and the dashed line represents the substance other than the analyte.

As described above, in the primary reaction, specific binding, nonspecific adsorption, and dissociation reach equilibrium. Removing the sample and introducing an analyte-free rinse solution (buffer) or the like to inside channel 41, however, shifts equilibrium toward dissociation. During this operation, as illustrated in FIG. 3, the magnitude of dissociation rate due to nonspecific adsorption and the magnitude of dissociation rate due to specific binding are different. The dissociation of the nonspecifically adsorbed substance other than the analyte progresses faster than the dissociation of the specifically and strongly bound analyte. Thus, a reduction rate in quantity of light for fluorescence γ due to the substance other than the analyte is larger than that due to the analyte (see FIG. 3). Accordingly, information about the reliability of the first signal value can be obtained by comparing a predetermined threshold value (described hereinafter) and a calculated reduction rate in quantity of light for fluorescence γ. This means, whether the reliability of the detected first signal value is high or low can be judged.

From the viewpoint of accurately obtaining information about the reliability of the first signal value, it is preferable to detect the first signal value and the second signal value so that a reduction rate in quantity of light for fluorescence γ becomes large. As illustrated in FIG. 3, in both cases of nonspecific adsorption and specific binding, dissociation progresses markedly immediately after equilibrium starts to shift, and progresses more gradually over time. Thus, the difference between reduction rates in quantity of light for fluorescence γ emitted from a fluorescent substance that labels the analyte and that emitted from a fluorescent substance that labels the substance other than the analyte becomes largest immediately after equilibrium starts to shift. It is preferable to detect signal values when a signal due to an analyte and signals due to substances other than the analyte can be distinguished more reliably. Accordingly, it is preferable to detect the first signal value within 3 minutes, more preferably within 30 seconds, after a signal becomes detectable. This is because, when the detection of the first signal value is delayed, distinguishing between a signal derived from a specifically binding analyte and a signal derived from a nonspecifically adsorbing substance other than the analyte becomes difficult. Further, an interval between obtaining the first signal value and obtaining the second signal value is preferably 20 to 60 seconds. The interval of 20 seconds or longer can increase a difference between reduction rates in quantity of light for fluorescence γ emitted from a fluorescent substance that labels an analyte and that emitted from a fluorescent substance that labels a substance other than the analyte, thereby obtain information about the reliability of the first signal value more accurately. In contrast, the interval of longer than 60 seconds is detrimental due to small changes in quantity of light for fluorescence γ, resulting in rather longer detection time.

The threshold value is a boundary value to distinguish a change rate of signals due to specific binding of an analyte from that due to nonspecific adsorption of a substance other than the analyte. The threshold value is not limited, and is appropriately adjusted depending on the type of a capturing body, the type of an analyte, the type of a sample, detection conditions of signals, or the like. A method for determining the threshold value is not limited. For example, the threshold value can be determined by performing, in advance, steps S 10 to S 60 and step S 80 using a sample containing only an analyte or a sample containing a substance other than the analyte, obtaining the respective change rates of signal values, and comparing the respective signal values.

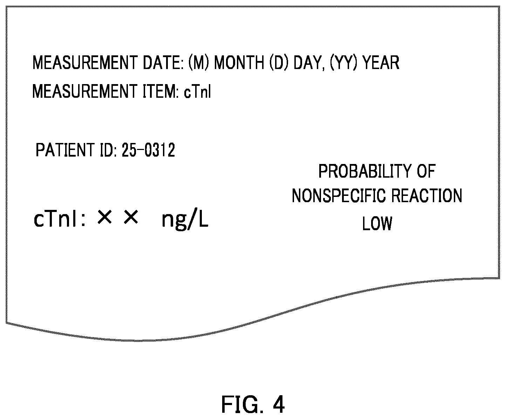

Through the above procedure, the reliability of the first signal value can be evaluated while the presence of an analyte in a sample is confirmed or the amount is determined. Further, FIG. 4 is a schematic view showing an example of the content to be output in an output section. The data shown in FIG. 4, such as a change rate of signal values or information about the reliability of the first signal value, obtained through the above procedure, is transmitted from control section 160 to output section 170, and is automatically output in output section 170. Control section 160 may convert as needed the first signal value to the concentration of the analyte or the like.

### [Effect]

As described in the foregoing, SPFS apparatus 100 according to the embodiment detects a signal generated in a reaction site twice, calculates a change rate of signal values, and obtains information about the reliability of a first signal value from the calculated change rate of signal values. This enables SPFS apparatus 100 to detect an analyte and judge whether detected signals are derived from an analyte or not in a short time and at a low cost without adding a new apparatus.

Although a signal is detected twice to calculate a change rate of signal values in the embodiment, a signal may be detected twice or more in the detection apparatus and the detection method of the present invention. In such a case, as long as a second signal value is detected after a first signal value, the first signal value and the second signal value can be obtained at any time of signal detection.

Further, although the detection apparatus and the detection method based on SPFS is described in the above embodiment, the detection apparatus and the detection method of the present invention is not limited to a detection method and a detection apparatus based on SPFS. For example, the detection apparatus and the detection method of the present invention may be a detection apparatus and a detection method based on SPR method. In this case, the detection apparatus detects quantity of light for reflected light as a signal value without measuring quantity of light for fluorescence. Accordingly, the secondary reaction (step S 40) is not needed.

### Examples

Hereinafter, the present invention will be described more specifically with reference to Examples, but the present invention is never limited to Examples below.

### 1. Determination of a threshold value

### (1) Preparation of a detection chip

A detection chip, in which anti-cardiac troponin I (cTnI) antibody, as a capturing body, is immobilized in a reaction site above a metal film, was prepared. The prepared detection chip was installed in a chip holder of a SPFS apparatus.

### (2) Primary reaction

Either a sample containing cardiac troponin I (cTnI) collected from a patient with heart disease (simply referred to as "cTnI sample" hereinafter) (50 µL) or a sample containing human anti-mouse IgG antibody (HAMA) that adsorbs to cTnI antibody nonspecifically in sufficient excess to endogenous cTnI (simply referred to as "HAMA sample" hereinafter) (50 µL) was provided to the reaction site using a syringe pump in a liquid feed section, and a primary reaction was performed while moving the liquid reciprocally inside a channel at a flow rate of 3000 µL/min for 5 minutes.

### (3) Measurement of an optical blank value

After the primary reaction, the sample inside the channel was removed and inside the channel was washed with a phosphoric acid buffer using the syringe pump in the liquid feed section. Then, excitation light (wavelength 635 nm) was irradiated on the metal film from the prism side. An optical blank value was obtained by detecting light emitted from the reaction site.

### (4) Secondary reaction

Then, a secondary reaction was performed by feeding a solution (60 µL) containing anti-cTnI antibody labeled with Alexa-Fluor (trademark) as a fluorophore, and moving the liquid reciprocally inside the channel using the syringe pump in the liquid feed section at a flow rate of 3000 µL/min for 3 minutes.

### (5) Detection of a first signal value

After the secondary reaction, the solution inside the channel was removed and inside the channel was washed with a phosphoric acid buffer using the syringe pump in the liquid feed section. In the same manner as the measurement of the optical blank value, within 10 seconds after the washing, fluorescence emitted from the reaction site was detected upon irradiation of excitation light. The first signal value was obtained by subtracting the optical blank value from the detected value in a processing section.

### (6) Detection of a second signal value

In the same manner as the detection of the first signal value, 30 seconds after the detection of the first signal value, fluorescence emitted from the reaction site was detected upon irradiation of excitation light. The second signal value was obtained by subtracting the optical blank value from the detected value in the processing section.

### (7) Calculation of a change rate of signal values and determination of a threshold value

A reduction rate in quantity of light for fluorescence (change rate of signal values) was calculated in the control section based on the first signal value and the second signal value. As cTnI samples, the above steps were performed for 139 samples. Further, as HAMA samples, the above steps were performed for 4 samples.

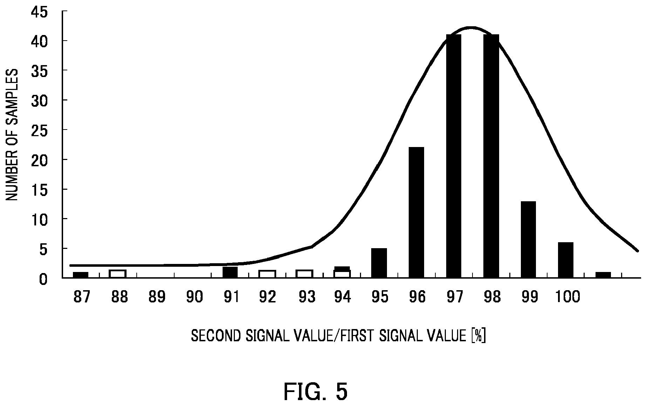

FIG. 5 is a histogram illustrating the distribution of reduction rates in quantity of light for fluorescence when signal values were detected for cTnI samples and HAMA samples. In FIG. 5, the black bars show the results for cTnI samples, and the white bars show the results for HAMA samples. The horizontal axis represents a quantity of light ratio [%] of the second signal value to the first signal value, and the vertical axis represents the number of samples. Comparing the results for cTnI samples with the results for HAMA samples in FIG. 5, reduction rates in quantity of light for fluorescence for cTnI samples are small, and the majority of quantity of light ratio of the second signal value to the first signal value is 95% or higher. In contrast, for HAMA samples, quantity of light ratio of the second signal value to the first signal value is less than 95%. Accordingly, it is preferable to set a threshold value to between 94 and 95% in the present Examples. In this case, as for the first signal value, when a calculated change rate of signal values is higher than or equal to a determined threshold value, the reliability can be judged to be high, whereas when a calculated change rate of signal values is lower than the determined threshold value, the false positive is likely and the reliability can be judged to be low.

### 2. Comparison between change rates of signal values

In each case of a cTnI sample and a HAMA sample, signal values were detected 10 times at a predetermined interval, and differences between reduction rates in quantity of light for fluorescence were investigated.

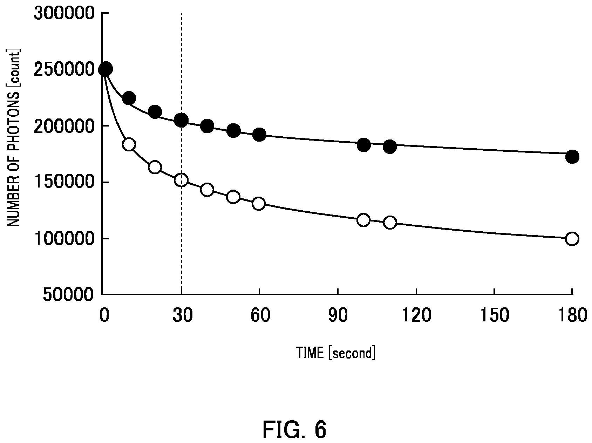

FIG. 6 is a graph illustrating differences between reduction rates in quantity of light for fluorescence when signal values were detected for a cTnI sample and a HAMA sample. In FIG. 6, the black dots are the results for a cTnI sample and indicate a reduction rate in quantity of light for fluorescence from a fluorescent substance that labels the specifically bound analyte. The white dots are results for a HAMA sample and indicate a reduction rate in quantity of light for fluorescence from a fluorescent substance that labels the nonspecifically adsorbed substance other than the analyte. In FIG. 6, the horizontal axis represents time [second] after a signal became detectable, and the vertical axis represents the number of photons [count] measured with a photomultiplier tube (PMT).

From the results for a cTnI sample and a HAMA sample in FIG. 6, a reduction rate in quantity of light for fluorescence was found to be smaller for a cTnI sample. Also, for both the samples, the signal values were found to decrease significantly within 30 seconds after a signal became detectable, and then the reduction rates in quantity of light for fluorescence became smaller over time.

From the above results, change rates of signal values were found to be different between the case of specific binding and the case of nonspecific adsorption. Accordingly, whether signals are derived from an analyte or from a substance other than the analyte can be judged by comparing a change rate of signal values with a predetermined threshold value. In such a case, from the viewpoint of increasing differences between change rates of signal values in the case of specific binding and in the case of nonspecific adsorption, the first signal value is preferably detected within 3 minutes, more preferably within 30 seconds after a signal becomes detectable.

This application is entitled to and claims the benefit of Japanese Patent Application No.2014-227031, filed on November 7, 2014, the disclosure of which including the specification, drawings and abstract is incorporated herein by reference in its entirety.

### Industrial Applicability

The detection apparatus and the detection method of an analyte according to the present invention can detect an analyte with high reliability, and thus are useful for tests for diseases, for example.

### Reference Signs List

- 10: Detection chip
- 20: Prism
- 21: Incident surface
- 22: Film forming surface
- 23: Emission surface
- 30: Metal film
- 40: Channel lid
- 41: Channel
- 100: SPFS apparatus
- 110: Light irradiation section
- 111: Light source unit
- 112: Angle adjustment mechanism
- 113: Light source control section
- 120: Reflected light detection section
- 121: Light receiving sensor
- 122: Angle adjustment mechanism
- 123: Sensor control section
- 130: Signal detection section
- 131: Light receiving unit
- 132: Position switching mechanism
- 133: Sensor control section
- 134: First lens
- 135: Optical filter
- 136: Second lens
- 137: Light receiving sensor
- 140: Liquid feed section
- 141: Liquid chip
- 142: Syringe pump
- 143: Liquid feed pump driving mechanism
- 144: Syringe
- 145: Plunger
- 150: Conveyance section
- 151: Conveyance stage
- 152: Chip holder
- 160: Control section
- 170: Output section
- α: Excitation light
- β: Reflected light
- γ: Fluorescence
- δ: Plasmon scattered light

## Claims

1. A detection apparatus configured to detect an analyte in a sample using a detection chip having a reaction site where a capturing body is immobilized, the apparatus comprising:
a holder for holding the detection chip;
a light irradiation section configured to irradiate the detection chip held by the holder with light;
a signal detection section configured to detect at least twice a signal generated in the reaction site when the light irradiation section irradiates the detection chip with light; and
a processing section configured to calculate a change rate of signal values based on a first signal value detected by the signal detection section and a second signal value detected after the first signal value.

2. The detection apparatus according to claim 1, wherein the processing section obtains information about reliability of the first signal value by comparing the change rate of signal values with a predetermined threshold value.

3. The detection apparatus according to claim 2, wherein the information about the reliability of the first signal value is information about an effect of noise due to a substance other than the analyte adsorbed in the reaction site nonspecifically.

4. The detection apparatus according to any one of claims 1 to 3, wherein the signal detection section detects the first signal value within 3 minutes after the signal becomes detectable.

5. The detection apparatus according to any one of claims 1 to 3, wherein the signal detection section detects the first signal value within 30 seconds after the signal becomes detectable.

6. The detection apparatus according to any one of claims 1 to 5, wherein an interval between detection of the first signal value and the second signal value in the signal detection section falls within a range of 20 to 60 seconds.

7. The detection apparatus according to any one of claims 1 to 6, wherein the number of times that the signal detection section detects a signal in order to calculate the change rate of signal values is two.

8. The detection apparatus according to any one of claims 1 to 7, further comprising an output section configured to output the change rate of signal values or information obtained using the change rate of signal values.

9. The detection apparatus according to any one of claims 1 to 8, wherein the signal detection section detects fluorescence from a fluorescent substance that labels the analyte captured by the capturing body, and
the processing section calculates a reduction rate in quantity of light for the fluorescence as the change rate of signal values.

10. The detection apparatus according to claim 9, wherein the detection chip includes a prism being a dielectric, a metal film disposed on the prism, and the capturing body immobilized in a region of the metal film that becomes the reaction site; and
the light irradiation section irradiates the metal film with light so as to generate surface plasmon resonance.

11. A detection method for detecting an analyte in a sample using a detection chip having a reaction site where a capturing body is immobilized, the method comprising:
providing the sample to the reaction site of the detection chip;
obtaining a first signal value by irradiating the detection chip with light in a state in which the sample provided to the reaction site is removed, and detecting a signal generated in the reaction site;
obtaining a second signal value after obtaining the first signal value by irradiating the detection chip with light and detecting a signal generated in the reaction site; and
calculating a change rate of signal values based on the first signal value and the second signal value.

12. The detection method according to claim 11, further comprising obtaining information about reliability of the first signal value by comparing the change rate of signal values with a predetermined threshold value.

13. The detection method according to claim 12, wherein the information about the reliability of the first signal value is information about an effect of noise due to a substance other than the analyte adsorbed on the reaction site nonspecifically.

14. The detection method according to any one of claims 11 to 13, wherein the obtaining of the first signal value includes detecting the signal within 3 minutes after the signal becomes detectable.

15. The detection method according to any one of the claims 11 to 13, the obtaining of the first signal value includes detecting the signal within 30 seconds after the signal becomes detectable.

16. The detection method according to any one of claims 11 to 15, wherein an interval between the obtaining of the first signal value and the obtaining of the second signal value falls within a range of 20 to 60 seconds.

17. The detection method according to any one of claims 11 to 16, wherein the number of times that the signal is detected in order to calculate the change rate of signal values is two.

18. The detection method according to any one of claims 11 to 17, wherein in the obtaining of the first signal value and the obtaining of the second signal value, the signal generated in the reaction site is quantity of light for fluorescence from a fluorescent substance that labels the analyte captured by the capturing body; and
in the calculating of the change rate of signal values, the change rate of signal values to be calculated is a reduction rate in quantity of light for the fluorescence.

19. The detection method according to any one of claims 11 to 18, wherein the detection chip includes a prism being a dielectric, a metal film disposed on the prism, and the capturing body immobilized in a region of the metal film that becomes the reaction site; and
the obtaining of the first signal value and the second signal value includes irradiating the metal film with light so as to generate surface plasmon resonance.
